# EUROPÄISCHE PATENTSCHRIFT

(11) **EP 0 764 148 B1**
(45) Veröffentlichungstag und Bekanntmachung des Hinweises auf die Patenterteilung: **21.07.1999**
(21) Anmeldenummer: 95923211.7
(22) Anmeldetag: 30.05.1995
(51) Int. Cl.: C07C 253/30, C07C 255/23, C09J 4/04

(54) **VERFAHREN ZUR HERSTELLUNG VON BISCYANOACRYLATEN**
PROCESS FOR PREPARING BISCYANOACRYLATES
PROCEDE DE PREPARATION DE BISCYANOACRYLATES

(30) Priorität: 06.06.1994 DE 4419740
(43) Veröffentlichungstag der Anmeldung: 26.03.1997
(73) Patentinhaber: Henkel Kommanditgesellschaft auf Aktien, 40191 Düsseldorf (DE)
(72) Erfinder: GOLOLOBOV, Yuri, Moscow, 117813 (RU); GRUBER, Werner, D-41352 Korschenbroich (DE); NICOLAISEN, Christian, D-30952 Ronnenberg (DE)
(86) Internationale Anmeldenummer: EP9502049
(87) Internationale Veröffentlichungsnummer: WO9533708

(56) Entgegenhaltungen:
- WO-A-94/15907
- DE-A- 1 764 554
- DE-A- 2 231 561
- US-A- 3 975 422
- RUSSIAN CHEMICAL BULLETIN, Bd.42, Nr.5, 1993 Seite 961 YU. GOLOLOBOV 'A novel approach to the synthesis of bis(2-cyanoacrylates)'
- DATABASE WPI Week 8046, Derwent Publications Ltd., London, GB; AN 80-82239C & SU,A,726 086 (HETEROORG. CPDS AS USSR)

## Beschreibung

Die Erfindung betrifft ein Verfahren zur Herstellung von Biscyanoacrylaten und deren Verwendung in Cyanoacrylat-Klebstoffen.

Biscyanoacrylate und ihre Herstellung sind seit langem bekannt. Zumindest gibt es folgende zwei Herstellungsmethoden:
- Bei der Knoevenagel-Kondensation werden Formaldehyd und Biscyanoacetate umgesetzt. Dabei entsteht ein vernetztes Polymer, welches kaum noch thermisch depolymerisiert werden kann.
- In der Retro-Diels-Alder-Reaktion wird zunächst ein monofunktionelles Cyanoacrylat mit Dienen blockiert. Das blockierte monofunktionelle Cyanoacrylat wird zur freien Säure verseift. Aus dem entsprechenden Säurechlorid wird mit einem Diol dann der Ester hergestellt. Nach dem Austausch des Biscyanoacrylates gegen Maleinsäureanhydrid wird schließlich nach wiederholtem Umkristallisieren aus Benzol das reine Biscyanoacrylat erhalten. Dieser Herstellungsweg umfaßt also 5 Stufen und ist daher unwirtschaftlich.

So beschreibt die US-A-3 975 422 ein Herstellverfahren für Biscyanoacrylaten durch Reaktion eines konjugierten Diens, beispielsweise Anthracen, mit einem Ester der 2-Cyanacrylsäure unter Bildung eines Diels-Alder-Adduktes des Esters. Anschließend wird dieses Esteraddukt hydrolisiert um letztendlich entweder das Diels-Alder-Addukt eines Alkalisalzes der 2-Cyanacrylsäure oder des 2-Cyanoacrylsäurehalogenids zu erhalten. Die letztgenannten Zwischenprodukte werden dann entweder mit einem Dihalogenid oder Diol der allgemeinen Formel X-R-X- umgesetzt. Dabei ist X entweder Chlor, Brom, Jod oder eine Hydroxygruppe und R ist ein zweiwertiger Alkylrest. Die Dien-Schutzgruppe kann dann entfernt werden beispielsweise durch Umsatz dieses letztgenannten Reaktionsproduktes mit einem Überschuß an Maleinsäureanhydrid. Anschließend muß das difunktionelle Cyanacrylat-Monomer isoliert werden. Die US-A-3 975 422 lehrt weiterhin, daß bestimmte Mono-Cyanacrylate wie z.B. Isobutyl-2-Cyanoacrylat mit den vorgeschilderten difunktionellen Cyanoacrylaten gemischt werden können. Die Polymerisation dieser Mischungen muß jedoch durch den Zusatz von basischen Katalysatoren wie Metallhydroxiden, basischen Salzen oder organischen Aminen initiiert werden. Lagerstabile Cyanoacrylat-Klebstoffe enthaltend Mischungen aus MonoCyanoacrylaten und Bis-Cyanaoacrylaten werden nicht offenbart.

Y. G. Gololobov und I. V. Chernoglazova beschreiben in Russ. Chem. Bull. Bd. 42, Nr. 5, 1993 Seite 961 ein Verfahren zur Herstellung von Bis(2-Cyanoacrylaten) ausgehend von der schwer zugänglichen freien Cyanacrylsäure und Diolen in einer ganz normalen Veresterungsreaktion. Konkret genannt werden 1,6-Hexandiol-Bis(2-Cyanacrylat) sowie das entsprechende 1,8-Octandiol-Derviat mit Ausbeuten von 32 %. Ein Verfahren, ausgehend von den leichter zugänglichen Monocyanacrylsäureestern wird nicht genannt. Weiterhin werden keine Mischungen von Biscyanoacrylaten mit Monocyanacrylat-Verbindungen offenbart.

Es besteht daher ein Bedarf nach einem einfachen Herstellungsweg für reines Biscyanoacrylat.

Die erfindungsgemäße Lösung ist den Ansprüchen zu entnehmen. Sie besteht im wesentlichen in der Umesterung von Monocyanoacrylaten mit Diolen sowie der anschließenden Aufarbeitung des Reaktionsgemisches durch fraktionierte Kristallisation. Das erfindungsgemäße Verfahren zur Herstellung von Biscyanoacrylaten besteht also darin, daß man 2-Cyanoacrylsäure oder deren Alkylester der allgemeinen Formel

H₂C = C(CN)-CO-O-R² (II)

worin R² ein verzweigter oder unverzweigter Alkylrest mit 1 bis 6 C-Atomen ist, mit Diolen der allgemeinen Formel

[HO]₂R¹ (III)

wobei R¹ ein verzweigter oder unverzweigter zweiwertiger Alkan-Rest mit 2 bis 18 C-Atomen ist, der auch noch Heteroatome wie Halogene und Sauerstoff oder aliphatische oder aromatische Ringe enthalten kann, zu Biscyanoacrylate der allgemeinen Formel

[H₂C = C(CN)-CO-O]₂R¹ (I)

umestert und dann das Reaktionsgemisch durch fraktionierte Kristallisation reinigt.

Ein Ausgangsprodukt ist also die monofunktionelle Cyanoacrylsäure oder deren Alkylester gemäß der Formel II. Der Alkylrest ist so zu wählen, daß der entstehende Alkohol leicht entfernt werden kann. Die dazu geeigneten Möglichkeiten sind dem Fachmann aus der allgemeinen Umesterungsreaktion bekannt. Vorzugsweise wird der Alkohol destillativ entfernt. Daher ist R² ein verzweigter oder unverzweigter Alkoholrest mit 1 bis 6 C-Atomen, vorzugsweise mit einem oder zwei C-Atomen. Der monofunktionelle Cyanoacrylsäureester ist wie üblich stabilisiert.

Bei den Diolen handelt es sich um zweiwertige primäre oder sekundäre Alkohole, vorzugsweise um primäre Alkohole. Die Hydroxylgruppen können zueinander in beliebiger Stellung stehen, vorzugsweise jedoch in Alpha/Omega-Stellung. Die Diole enthalten 2 bis 18 C-Atome, vorzugsweise 4 bis 12 C-Atome. Sie können linear, verzweigt oder zyklisch angeordnet sein. Der aliphatische Rest kann auch eine aromatische Gruppe enthalten oder neben den Wasserstoff- und Kohlenstoffatomen auch noch Heteroatome, wie z.B. Chlor- oder Sauerstoff-Atome, vorzugsweise in Form von Polyethylen- oder Polypropylenglykoleinheiten. Als konkrete Diole seien genannt: Hexandiol, Octandiol, Dekandiol und Dodecandiol.

Der Cyanoacrylsäureester wird im Überschuß eingesetzt. Das molare Verhältnis von monofunktionellem Cyanoacrylsäureester zum Diol beträgt also mindestens 2,0 : 1,0, vorzugsweise jedoch 2,5 : 1,0, insbesondere 2,2 : 1,0.

Die Umesterung wird durch starke Stäuren katalysiert, insbesondere durch Sulfonsäuren, vorzugsweise durch aromatische Sulfonsäuren, wie z.B. p-Toluolsulfonsäure. Aber auch Naphthalinsulfonsäure und Benzolsulfonsäure sowie saure Ionenaustauscher sind möglich. Die Konzentration des Umesterungskatalysators sollte zwischen 1 und 20 Gew.-% liegen, bezogen auf das monofunktionelle Cyanoacrylat.

Die Umesterung erfolgt - wie auch sonst üblich - in Lösung. Als Lösungsmittel dienen Aromaten und Halogenkohlenwasserstoffe. Bevorzugtes Lösungsmittel ist Toluol und Xylol. Die Konzentration der Lösung liegt im Bereich von 10 bis 50, vorzugsweise von 10 bis 20 %.

Der entstehende einwertige Alkohol bzw. das entstehende Wasser werden auf bekannte Art und Weise entfernt, vorzugsweise mit dem Lösungsmittel abdestilliert. Der Umsatz der Umesterung wird kontrolliert z.B. anhand von NMR-Spektren. Wie auch sonst dauert die Reaktion mehrere Stunden. Im Falle von Toluol als Lösungsmittel und p-Toluolsulfonsäure als Katalysator ist die Reaktion nach 10 bis 15 Stunden beendet, d.h. es scheidet sich kein Alkohol mehr ab.

Sehr wichtig ist nun die Aufarbeitung des Reaktionsgemisches. Im Falle von sauren Ionenaustauschern als Katalysator können diese einfach abfiltriert werden. Im Falle von löslichen Sulfonsäuren als Katalysator z.B. von p-Toluolsulfonsäure wird diese durch Lösungsmittelsubstitution abgetrennt: Toluol wird gegen eine Mischung aus Hexan, Heptan oder Dekan ersetzt. Nach zweimaliger fraktionierter Kristallisation erhält man reines Biscyanoacrylat. Die Reinheit beträgt nach NMR-Spektren mehr als 99 %.

Das erhaltene Biscyanoacrylat ist mit den üblichen Stabilisatoren und in den üblichen Konzentrationen lagerstabil, d.h. es verändert bei 20 °C innerhalb von 6 Monaten seinen Schmelzpunkt praktisch nicht.

Die erhaltenen Biscyanoacrylate polymerisieren aber in Gegenwart von Basen sehr schnell. Wie bei den monofunktionellen Cyanoacrylaten reichen Spuren von Wasser bereits aus. Es entsteht dann ein dreidimensional vernetztes Polymer mit relativ guten thermischen Eigenschaften.

Vorzugsweise wird es daher in bekannten Cyanoacrylat-Klebstoffen mitverwendet und zwar in einer Menge von 1 bis 50, vorzugsweise von 2 bis 10 Gew.-%, bezogen auf den Klebstoff insgesamt.

Die bekannten Cyanoacrylatklebstoffe enthalten als Hauptkomponente 2-Cyanoacrylsäureester der allgemeinen Formel

H₂C = C(CN)-CO-O-R (IV).

In ihr ist R eine Alkyl-, Alkenyl-, Cycloalkyl-, Aryl-, Alkoxyalkyl-, Aralkyl- oder Haloalkylgruppe, insbesondere eine Methyl-, Ethyl-, n-Propyl-, iso-Propyl-, n-Butyl-, iso-Butyl-, Pentyl-, Hexyl-, Allyl-, Methallyl-, Crotyl-, Propargyl-, Cyclohexyl-, Benzyl-, Phenyl-, Cresyl-, 2-Chlorethyl-, 3-Chlorpropyl-, 2-Chlorbutyl-, Trifluorethyl-, 2-Methoxyethyl-, 3-Methoxybutyl- und 2-Ethoxyethylgruppe. Die vorgenannten Cyanoacrylate sind dem Klebstoffachmann bekannt, vgl. Ullmanns's Encyclopaedia of Industrial Chemystry, Bd. A1, S. 240, Verlag Chemie Weinheim (1985) sowie US-PS 3 254 111 und PS-PS 3 654 340. Bevorzugte Monomere sind die Allyl-, Methoxyethyl-, Ethoxyethyl-, Methyl-, Ethyl-, Propyl- oder Butyl-Ester der 2-Cyanoacrylsäure.

Der Klebstoff kann Additive enthalten z.B. Weichmacher, Verdicker, Stabilisatoren, Aktivatoren, Farbstoffe usw.

Der erfindungsgemäße neue Cyanoacrylatklebstoff eignet sich besonders für Verklebungen mit hohen thermischen Anforderungen z.B. zum Verkleben von elektrischen und elektronischen Bauteilen.

Die Erfindung wird nun anhand von Beispielen im einzelnen erläutert:

### I. Herstellung von Biscyanoacrylaten

Im Rahmen des vorbeschriebenen allgemeinen Herstellungsverfahrens wurden die in der Tabelle 1 angegebenen Ausgangsprodukte in 1 Kg Toluol mit p-Toluol-Sulfonsäure als Katalysator umgesetzt. Nach 6 Stunden war die Umesterung beendet. Das Toluol wurde nun durch Hexan ersetzt. Nach zweimaliger fraktionierter Kristallisation wurden die entsprechenden Biscyanoacrylate mit denen in der Tabelle angegebenen Schmelzpunkten erhalten.

**Tabelle 1**

| lfd. Nr. | Ausgangsprodukte | a) 1,0 : 0,5 Ansatz g | b) 1,2 : 0,4 Ansatz g | Schmelzpunkt °C |
|---|---|---|---|---|
| 1. | Cyanacrylsäuremethylester | 65,96 | 69,99 | 59-60 |
| | 1,6-Hexandiol | 35,05 | 27,68 | |
| 2. | Cyanacrylsäuremethylester | 60,92 | 65,24 | 65-67 |
| | 1,8-Octandiol | 40,08 | 35,76 | |
| 3. | Cyanacrylsäuremethylester | 56,63 | 61,10 | 74-75 |
| | 1,10-Decandiol | 44,38 | 39,91 | |
| 4. | Cyanacrylsäuremethylester | 52,89 | 57,45 | 79-80 |
| | 1,12-Dodecandiol | 48,12 | 43,56 | |

### II. Verwendung der Biscyanoacrylate in Cyanoacrylaten-Klebstoffen

Auf die gereinigten (gestrahlten) Aluminium- oder Stahl-Bleche wurden einige Tropfen des Cyanoacrylatklebstoffes auf der Basis von Cyanoacrylsäureethylester mit den angegebenen Zusätzen an Biscyanoacrylaten gegeben und bei 20 °C in 24 Stunden ausgehärtet. Danach wurden die verklebten Bleche bei 20, 100 und 150 °C 3 Tage gelagert und bei diesen Temperaturen auf Festigkeit geprüft.

**Tabelle 2:**

| Zugscherfestigkeit (m N/mm²) | | | | | | | |
|---|---|---|---|---|---|---|---|
| lfd. Nr. | | Biscyanoacrylate | | Substrat | Zugscherfestigkeit | | |
| | | Art | Menge [%] | | 20 °C | 100 °C | 150 °C |
| 1 | a) | - | 0 | Stahl | | 5 | 3 |
| | b) | - | 0 | Al | | 6 | 2 |
| 2 | | Hexandiol-Biscyanoacrylat | 5 | Stahl | 21 | 18 | 12 |
| 3 | | Octandiol | 10 | Al | 18 | 16 | 10 |

## Patentansprüche

1. Verfahren zur Herstellung von Biscyanoacrylaten der allgemeinen Formel
[H₂C = C(CN)-CO-O]₂R₁ (I)
wobei R¹ ein verzweigter oder unverzweigter zweiwertiger Alkan-Rest mit 2 bis 18 C-Atomen ist, der auch noch Heteroatome wie Halogen und Sauerstoff sowie aliphatischen oder aromatische Ringe enthalten kann, durch
a) Umesterung der 2-Cyanoacrylsäure oder deren Alkylester der allgemeinen Formel
H₂C = C(CN)-CO-O-R² (II)
wobei R₂ ein verzweigter oder unverzweigter Alkylrest mit 1 bis 6 C-Atomen ist, mit Diolen der allgemeinen Formel
[HO]₂R¹ (III)
und
b) Aufarbeitung des Reaktionsgemisches durch fraktionierte Kristallisation.

2. Verfahren nach Anspruch 1, gekennzeichnet durch die Umesterung in Gegenwart von Sulfonsäuren, insbesondere Toluolsulfonsäure in einer Konzentration von 1 bis 20 Gew.-%, bezogen auf das monofunktionelle Cyanoacrylat.

3. Verfahren nach Anspruch 1 oder 2, gekennzeichnet durch Toluol oder Xylol als Lösungsmittel für die Umesterung und Hexan, Heptan oder Dekan für die fraktionierte Kristallisation.

4. Lagerstabiler Cyanoacrylat-Klebstoff enthaltend 99 bis 50, vorzugsweise 98 bis 90 Gew.-% 2-Cyanoacrylsäureester der allgemeinen Formel
H₂C = C(CN)-CO-O-R (IV),
in der R eine Alkyl-, Alkenyl-, Cycloalkyl-, Aryl-, Alkoxyalkyl-, Aralkyl- oder Haloalkylgruppe, insbesondere eine Methyl-, Ethyl-, n-Propyl-, iso-Propyl-, n-Butyl-, iso-Butyl-, Pentyl-, Hexyl-, Allyl-, Methallyl-, Crotyl-, Propargyl-, Cyclohexyl-, Benzyl-, Phenyl-, Cresyl-, 2-Chlorethyl-, 3-Chlorpropyl-, 2-Chlorbutyl-, Trifluorethyl-, 2-Methoxyethyl-, 3-Methoxybutyl- oder 2-Ethoxyethylgruppe ist
sowie
1 bis 50, vorzugsweise 2 bis 10 Gew.-% eines Biscyanoacrylates gemäß Formel I sowie ggf. Weichmacher, Verdicker, Stabilisatoren, Aktivatoren und/oder Farbstoffe.

5. Cyanoacrylat-Klebstoff nach Anspruch 4, dadurch gekennzeichnet, daß R¹ 6, 8, 10 oder 12 C-Atome enthält.

6. Cyanoacrylat-Klebstoff nach Anspruch 4 oder 5 zum Verkleben von elektrischen und elektronischen Bauteilen.

## Claims

1. A process for the production of biscyanoacrylates corresponding to the following general formula:
[H₂C = C(CN)-CO-O]₂R¹ (I)
in which R¹ is a branched or unbranched difunctional alkane group containing 2 to 18 carbon atoms, which may also contain hetero atoms, such as halogen and oxygen, and aliphatic or aromatic rings,
characterized in that
a) 2-cyanoacrylic acid corresponding to the following general formula:
H₂C = C(CN)-CO-O-R² (II)
in which R² is a branched or unbranched alkyl group containing 1 to 6 carbon atoms,
or an alkyl ester thereof is transesterified with diols corresponding to the following general formula:
[HO]₂R¹ (III)
and
b) the reaction mixture is subsequently worked up by fractional crystallization.

2. A process as claimed in claim 1, characterized in that the transesterification is carried out in the presence of sulfonic acids, more particularly toluene sulfonic acid, in a concentration of 1 to 20% by weight, based on the monofunctional cyanoacrylate.

3. A process as claimed in claim 1 or 2, characterized in that toluene or xylene is used as the solvent for the transesterification and hexane, heptane or decane is used for the fractional crystallization.

4. A storage-stable cyanoacrylate adhesive containing 99 to 50% by weight and preferably 98 to 90% by weight of 2-cyanoacrylates corresponding to the following general formula:
H₂C = C(CN)-CO-O-R (IV)
in which R is an alkyl, alkenyl, cycloalkyl, aryl, alkoxyalkyl, aralkyl or haloalkyl group, more particularly a methyl, ethyl, n-propyl, isopropyl, n-butyl, isobutyl, pentyl, hexyl, allyl, methallyl, crotyl, propargyl, cyclohexyl, benzyl, phenyl, cresyl, 2-chloroethyl, 3-chloropropyl, 2-chlorobutyl, trifluoroethyl, 2-methoxyethyl, 3-methoxybutyl and 2-ethoxyethyl group,
and 1 to 50% by weight and preferably 2 to 10% by weight of a biscyanoacrylate corresponding to formula I and optionally plasticizers, thickeners, stabilizers, activators and/or dyes.

5. A cyanoacrylate adhesive as claimed in claim 4, characterized in that R¹ contains 6, 8, 10 or 12 carbon atoms.

6. A cyanoacrylate adhesive as claimed in claim 4 or 5 for bonding electrical and electronic components.

## Revendications

1. Procédé de fabrication de biscyanoacrylates de formule générale :
[H₂C = C(CN)-CO-O]₂R¹ (I)
dans laquelle R¹ est un reste alkane bivalent ramifié ou non ramifié, ayant de 2 à 18 atomes de carbone, qui peut contenir aussi encore des hétéroatomes ainsi que des cycles aliphatiques ou aromatiques par :
a) transestérification de l'acide 2-cyanoacrylique ou de ses esters d'alkyle de formule générale :
H₂C = C(CN)-CO-O-R² (II)
dans laquelle R² est un radical alkyle ramifié ou non ramifié, ayant de 1 à 6 atomes de carbone,
avec des diols de formule générale :
[HO]₂R¹ (III)
et,
b) traitement du mélange réactionnel par cristallisation fractionnée.

2. Procédé selon la revendication 1,
caractérisé par
la transestérification en présence d'acides sulfoniques, en particulier d'acide toluène sulfonique à une concentration allant de 1 à 20 % en poids, rapporté au cyanoacrylate monofonctionnel.

3. Procédé selon la revendication 1,
caractérisé par
l'utilisation de toluène ou de xylène comme solvant pour la transestérification et d'hexane, d'heptane ou de décane pour la cristallisation fractionnée.

4. Colle au cyanoacrylate stable au stockage renfermant de 99 à 50, de préférence de 98 à 90 % en poids d'ester d'acide 2-cyanoacrylique de formule générale :
H₂C = C(CN)-CO-O-R (IV)
dans laquelle R est un radical alkyle, alkényle, cycloalkyle, aryle, alkoxyalkyle, aralkyle ou un groupe haloalkyle, en particulier un groupe méthyle, éthyle, n-propyle, iso-propyle, n-butyle, iso-butyle, pentyle, hexyle, allyle, méthallyle, crotyle, propargyle, cyclohexyle, benzyle, phényle, crésyle, 2-chloréthyle, 3-chloropropyle, 2-chlorobutyle, Trifluoréthyle, 2-méthoxyéthyle, 3-méthoxybutyle ou 2-éthoxyéthyle ainsi que de 1 à 50, de préférence de 2 à 10 % en poids, d'un bis cyanoacrylate conformément à la formule I ainsi qu'éventuellement des agents plastifiants, des agents épaississants, des agents stabilisants, des activateurs et/ou des colorants.

5. Colle au cyanoacrylate selon la revendication 4,
caractérisée en ce que
R¹ contient 6, 8, 10 ou 12 atomes de carbone.

6. Colle au cyanoacrylate selon la revendication 4 ou 5, en vue de l'encollage d'éléments de construction électriques et électroniques.
